# EUROPEAN PATENT SPECIFICATION

(11) **EP 3 474 949 B1**
(45) Date of publication and mention of the grant of the patent: **13.05.2020**
(21) Application number: 17730500.0
(22) Date of filing: 20.06.2017
(51) Int. Cl.: A61N 5/06

(54) **THERMAL SAFEGUARD FOR LIGHT TREATMENT DEVICES**
THERMISCHER SCHUTZ FÜR LICHTBEHANDLUNGSVORRICHTUNGEN
PROTECTION THERMIQUE POUR DISPOSITIFS DE TRAITEMENT DE LUMIÈRE

(30) Priority: 23.06.2016 EP 16175982
(43) Date of publication of application: 01.05.2019
(73) Proprietor: Koninklijke Philips N.V., 5656 AG Eindhoven (NL)
(72) Inventor: DANKERS, Gerardus, Maria, 5656 AE Eindhoven (NL)
(74) Representative: Kapoor, Pavan Puneet
(86) International application number: PCT/EP2017/065014
(87) International publication number: WO 2017/220530

(56) References cited:
- WO-A1-2013/046086
- WO-A1-2014/024092
- WO-A1-2016/016792
- US-A1- 2012 116 485
- US-A1- 2016 114 184

## Description

### FIELD OF THE INVENTION

The present invention relates to phototherapy blankets, more specifically, the invention relates to thermal management in the phototherapy blanket.

### BACKGROUND OF THE INVENTION

A phototherapy blanket is known from US 2016/114184. phototherapy blankets include multiple light sources, preferably Light Emitting Diodes (LEDs), which provide optical energy for treating a patient/ subject. Typically, these phototherapy blankets are used in the field of treating neonatal hyperbilirubinemia (newborn jaundice). It is crucial to monitor temperature of a baby in these phototherapy blankets as optical energy is transformed into thermal energy, which can heat up the skin. The rise in skin temperature should not exceed a maximum limit. To check the skin temperature, currently, at least one temperature sensor is integrated in the phototherapy blanket that measures temperature in certain location(s)/ region(s) of the body. In current implementations, two temperature sensors are positioned in the blanket such that they lay below the skin of the newborn/baby. The two temperatures are positioned such that two different locations/ regions of the surface of the body part can be checked at any given point in time. If one of the temperature sensors records a temperature higher than a pre-determined maximum limit, a control system of the phototherapy blankets shuts OFF the light sources.

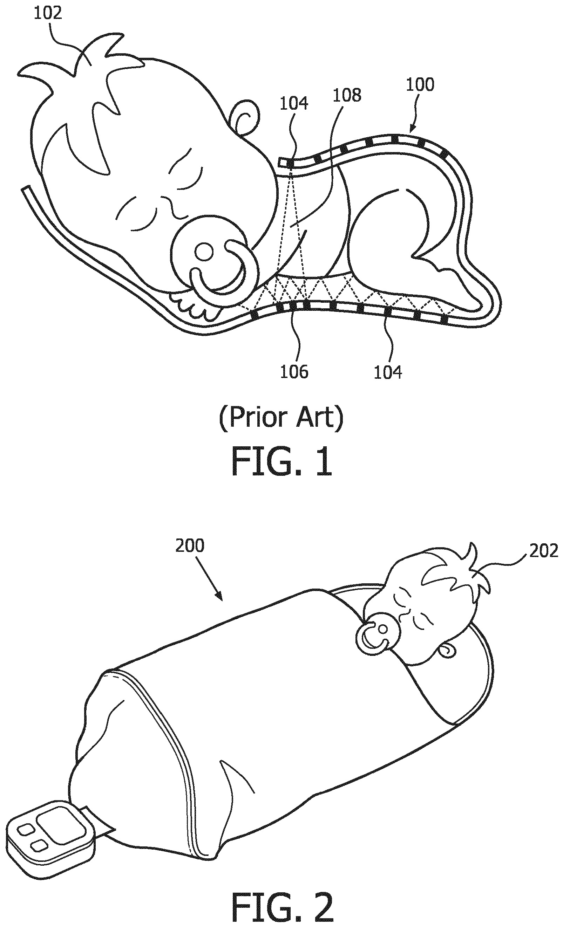

However, babies are not still throughout the treatment duration and there are chances that they move and thus do not directly lay above the temperature sensor. Therefore, the temperature sensed by the temperature sensor at times is not because of the skin temperature but is because of light sources integrated in the blanket. Thus, the control system incorrectly decides to shut off the light sources and interrupts the treatment prematurely, which is clearly undesired. This is also depicted in Fig. 1. Fig. 1 shows a phototherapy blanket 100 enveloping a baby 102 inside. The blanket 100 further includes LED sources 104 and a temperature sensor 106. As it can be seen from the figure, due to the position of the baby, a subset of LED sources 102 instead of emitting light (electromagnetic radiation) towards the skin of the baby, emit the radiation 108 towards the temperature sensor. In such a scenario, as explained above, the temperature sensor records the rise in temperature due to the optical energy and not because of the skin of the baby.

Thus, there remains a need for a temperature sensing arrangement which is easy to implement and also gives reliable results.

### SUMMARY OF THE INVENTION

The invention is defined by the claims.

A phototherapy blanket configured to cover, support, and/or envelop at least part of a subject, the phototherapy blanket comprising: a set of light sources configured to emit electromagnetic radiation, wherein the set of light sources is held and/or carried by the phototherapy blanket; a temperature sensor configured to generate a first signal conveying information related to a temperature of a region of a surface of the at least part of the subject covered, supported, and/or enveloped in the phototherapy blanket, a surface sensor configured to provide information about presence of the surface of the at least part of the subject, wherein the surface sensor is positioned proximate to the temperature sensor such that the surface sensor is configured to generate a second signal indicative of presence of the region of the surface of the at least part of the subject; and a control module configured to control the intensity of the set of light sources based on the first and the second signal.

As explained earlier, in the practical scenario, when the baby moves inside the blanket, at least one of the temperature sensors inside the blanket does not sense/ record the temperature of the region of the surface of the body part of the baby but records the temperature that is generated due to the thermal energy transformed from the optical energy of the light sources. In other words, the light hitting the temperature sensor generates the heat that is then sensed by the temperature sensor (also depicted in Fig. 1). The control system interprets this as the temperature of the baby. By using an additional surface sensor in proximity to the temperature sensor, the control system further checks if the rise in temperature detected is because of the presence of the region of the surface, preferably skin, of the body part of the baby. One can imagine that the positioning of the surface sensor in proximate to the temperature sensor is important as it helps the control module to decide if the temperature is indeed because of the region of the surface of the body part of the baby. In absence of the region detection, the control system continues to keep the state ON of the light sources and thus the treatment in continued. Clearly, the surface sensor provides additional intelligence in controlling, such as maintaining the ON state, or switching OFF, the light sources. As it can be appreciated by a person skilled in the art that with the invention as claimed in claims now avoids false positives and thus provides a reliable system for control system for temperature detection.

Phototherapy blanket term refers to any light treatment device that is capable to envelope/ cover/ support most of the body part of the subject, such that the light sources of the phototherapy blanket emit the electromagnetic radiations (either from all the sides or only from one direction) towards the subject or a body part of the subject. More specifically, the light sources carried/ integrated in the blanket (or light treatment device) face the temperature sensor, when the subject/ body part is not in the blanket or slides away from the temperature sensor. Light treatment device is further designed to form a space there between to receive the subject/ body part. For instance the phototherapy blanket can be a circular such that it can envelope a thigh of the subject. In most of the practical embodiments, the subject or the body part to be irradiated with light is positioned/ enveloped bare skin in the photo therapy blanket and thus, the surface inside the phototherapy blanket mostly refers to the bare skin of the subject/ body part of the subject.

In a further embodiment, the control module is configured to control the intensity of the light sources in the ON state. Based on the temperature sensed, the control unit can further control the intensity of the light sources in order to provide effective phototherapy. This is advantageous as the treatment can continue in the ON state but can be milder by reducing the intensity of the light sources or can be stronger by increasing the intensity of the light sources.

In a further embodiment, the control module is configured to determine if the temperature, i.e. the temperature of the region, recorded by the temperature sensor is above a temperature threshold, wherein when the temperature is above the temperature threshold, the control module is further configured to control the intensity of the set of light sources based on the second signal.

In a further embodiment, the control module is further configured to generate an alert for a caregiver of the subject, hereinafter referred to as a baby, in the OFF state. This is in particular advantageous as this helps the caregiver attend immediately to the need of the baby. For instance, the control system determines that the temperature increase is indeed because of the surface of the body part of the baby, which essentially means that the baby has high temperature. In such cases, the control system sends an alert signal to let the caregiver be aware of the situation so that the situation can tackled effectively. The term "caregiver" refers to any user that supports the baby, i.e. the subject, undergoing the treatment, for instance mother, friend, nurse, doctor, etc. Various examples of the alert signal may include but is not limited to an audio signal, a video signal, a tactile signal or a combination thereof.

In a further embodiment, the temperature sensor and surface sensor are placed in a pair, wherein the phototherapy blanket comprises at least two such sensor pairs, wherein when the control module determines that the temperature of the region is above a temperature threshold based on the first signal provided by a first temperature sensor of a first sensor pair of the at least two sensor pairs, the control module is further configured to control the intensity of the set of light sources based on the second signal provided by the corresponding first surface sensor of the first sensor pair. This further increases the reliability of the measurement.

In a further embodiment, the set of light sources are arranged in form of a matrix structure, wherein the matrix structure comprises rows and columns of light sources. It may be appreciated by a person skilled in the art that the light sources can be arranged in different forms, such as spiral, circular, etc.

In a further embodiment, the surface sensor is at least one of a photo resistor or a photodiode. In the advantageous embodiment, when the temperature of the region is above a temperature threshold, the control module is configured to check if the second signal that photo resistor or a photodiode (i.e. the surface sensors) outputs is indicative of light, wherein when the second signal indicates that light is detected, the second signal is indicative of non-detection of the region of the surface of the at least part of the subject.

In a further embodiment, the skin detecting sensor is one of a skin conductance sensor, a pressure sensor and a capacitive sensor. Unlike, the above sensors, i.e. photo resistor and photodiode/ photo detector, these sensors directly contact the skin of the subject. In the above sensors, the recording of the incident light is interpreted as non-detection of the region. However, with sensors, such as skin conductance sensor, pressure sensors, capacitive sensors, it is required that these sensors contact the skin (surface) of the subject in order to detect skin/presence of the region.

In a further embodiment, the light source is a Light Emitting Diode (LED), and wherein the control module is configured to switch at least one LED in proximate to the temperature sensor in a detection mode for a time-period, wherein, the LED in the detection mode is the surface sensor, wherein the LED in detection mode is a photodetector and the control module is further configured to check if the second signal that the photodetector outputs is indicative of light, wherein when the second signal indicates that light is detected, the second signal is indicative of non-detection of the region of the surface of the at least part of the subject.

In an embodiment of the invention, the temperature sensor is a thermistor, in particular a negative temperature coefficient (NTC) thermistor.

In another aspect a method to be performed by a control system of a phototherapy blanket is provided. The phototherapy blanket comprising a set of light sources, the method includes: receiving a first signal indicative of a temperature of a region of a surface of at least part of a subject; wherein the at least part of the subject is covered and/ or enveloped in the phototherapy blanket, wherein the first signal is provided by a temperature sensor included in the phototherapy blanket; receiving a second signal indicative of presence of the region of a surface of the at least part of the subject, wherein the second signal is provided by a surface sensor, wherein the surface sensor is positioned in proximate of the temperature sensor; and controlling the intensity of the set of light sources based on the first and the second signal. The method may be implemented at least in part in software.

In yet another aspect, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed. The computer program may be stored/distributed on a suitable medium, such as an optical storage medium or a solid-state medium supplied together with or as part of other hardware, but may also be distributed in other forms, such as via the Internet or other wired or wireless telecommunication systems. In particular, in an aspect, the computer program includes the program code means for implementing the steps of the method as disclosed herein is executed by a control system of the phototherapy blanket.

Preferred embodiments of the invention are defined in the dependent claims. It shall be understood that the claimed phototherapy blanket, computer program and medium can have similar and/or identical preferred embodiments as the claimed device, in particular as defined in the dependent claims and as disclosed herein.

### BRIEF DESCRIPTION OF THE DRAWINGS

Examples of the invention will now be described in detail with reference to the accompanying drawings, in which:
Figure 2 shows a phototherapy blanket covering/ enveloping the baby;
Figure 3a, 3b, and 3c show a phototherapy blanket according to an embodiment of the invention;
Figure 4 shows a method executed by a control module of the photo therapy blanket according to the disclosure; and
Figure 5 shows a phototherapy blanket according to another embodiment of the invention; and
Figure 6 shows a phototherapy blanket according to yet another embodiment of the invention.

### DETAILED DESCRIPTION OF THE EMBODIMENTS

The invention provides a phototherapy blanket with improved temperature detection.

Figure 2 shows a phototherapy blanket 200 covering/ enveloping the baby 202. As it can be seen from the figure, the idea of the blanket is to over the baby totally so that the whole body can be irradiated with electromagnetic radiation emitted by light sources (not shown) hits the subject directly and thereby provides treatment for newborn jaundice. For treatment of newborn jaundice blue light in the range of 460-490 nm is found to be most effective.

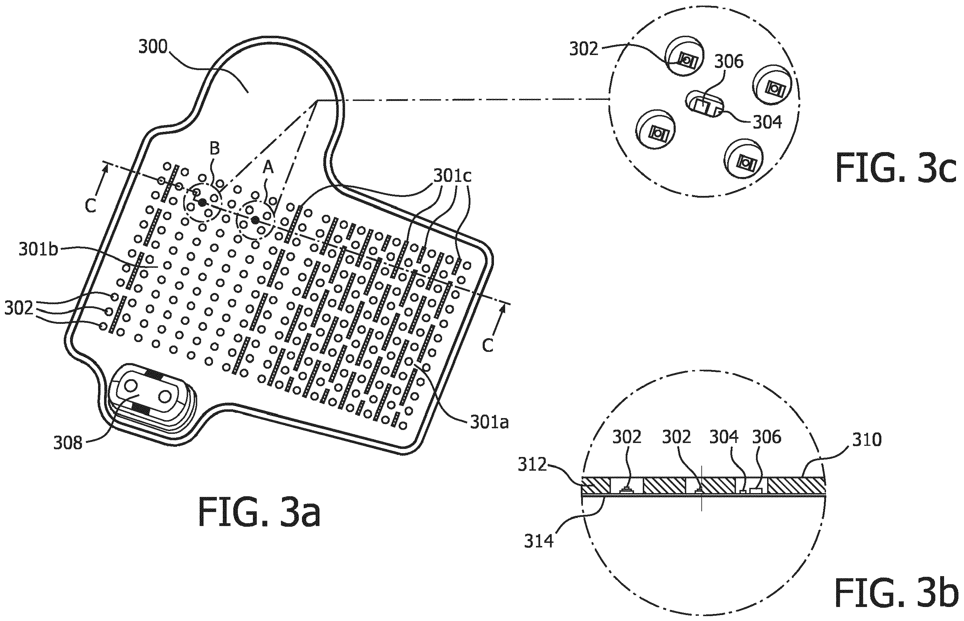

Figure 3a, 3b, and 3c shows a phototherapy blanket 300 according to an embodiment. The phototherapy blanket 300 includes a front surface 301a and a back surface 301b, a set of light sources 302 arranged in a matrix structure of rows and columns, two temperature sensors 304a and 304b, two surface sensors 306a and 306b and a control module 308. In the current embodiment, the light sources 302 are LEDs 302.

The back surface 301b is a surface on which the baby is supposed to lay with his back on. The front surface 301a is the surface that is wrapped around the baby's front portion (chest, abdomen, etc.) such that baby can be enveloped inside the blanket 300. Multiple lines 301c are called the folding lines to improve the flexibility of the folding the front 301a and back surface 301b respectively.

The blanket 300 is typically made of a flexible material. When seen along the section C-C, in Fig. 3b, the top layer 310 is a protective layer that rests upon a spacer fabric 312 that creates a height (space) between the electronic components, i.e. LEDs 302, temperature sensor 304, surface sensor 306, and the top layer 310. This is in particular designed so that the electronic components do not touch the baby directly. Secondly, a bottom layer 314 supports the spacer fabric 312 and the LEDs 302, the temperature sensor 304, and the surface sensor 306.

In an embodiment of the invention, the top layer 310 is thin flexible foil made of bio-compatible material which is optically transmissive polyester. Further, a disposable layer, such as woven thin layer, can be placed on top of the top layer 310. This increases hygiene and reusability of the blanket 300. Thus, in a hospital setup the same blanket 300 can be used by changing the top layer 310 for multiple babies.

Further, temperature sensor 304, such as a thermistor, and the surface sensor 306, such as a photo-resistor, are depicted in Fig. 3c. The two sensors are placed in a pair (also depicted in Fig. 3c). Two such sensor pairs are depicted by dotted circles in A and B. The first sensor pair A includes the first temperature sensor 304a and the first surface sensor 306a and the second pair B includes the second temperature sensor 304b and the second surface sensor 306b. The thermistors, in each pair, are placed such that when the baby is enveloped inside the blanket 300, the baby lays over the each of the thermistors 304 and thus each provides a first signal that is indicative of the temperature of the region of the surface of the baby facing the corresponding thermistor 304. Further, the two thermistors 304 are arranged to sense/record temperature at two different regions of the surface (also depicted in Fig 3a, dotted circles). Thermistors 304 are arranged such that they sense temperature of two distinct and non-overlapping regions. In other words, the temperature thus sensed is very local to the temperature of the region directly laying above the thermistor 304. For instance, in the current implementation, when the baby lays on the back surface 301b, the first thermistor will sense the temperature of left region of the back surface of the baby, while the second thermistor will sense the temperature of the right region of the back surface of the baby. As explained earlier, the term region is to be understood as the region directly facing (in front of) the thermistor.

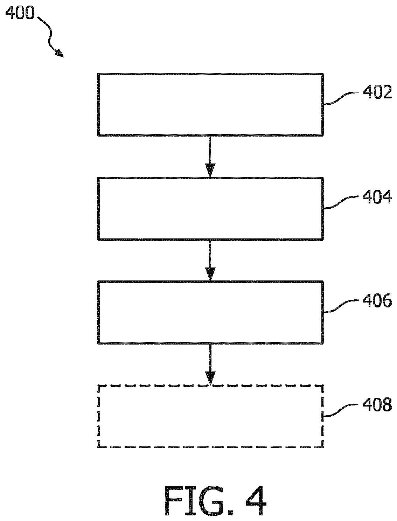

Furthermore, both the sensors (304, 306) are placed adjacent/ proximate to each other. The proximity is important to ensure that the temperature of the region thus sensed by the thermistor 304 is actually of the region (presence of which is sensed by surface sensor 306) that thermistor 304 is facing. In the current implementation, the proximity can be between 0.1 mm -10 mm. The proximity is also dependent on the size and the accuracy of the temperature sensor 304 and/ or the surface sensor 306. In an embodiment of the invention, the two sensors (304, 306) can be integrated on a common flexible PCB (printed circuit board). For the current embodiment, the surface sensor 306 is a photo-resistor 306. Further working of the two sensors to control, for instance, either determine to maintain the ON state or to switch OFF the LEDs 302 is explained in detail in conjunction with Fig. 4.

It may be appreciated that though the current set up is explained with two temperature sensors and two surface sensors (i.e. two pairs), the blanket 300 can be designed with one of each sensors only or can have more than two based on the accuracy requirements.

Fig. 4 shows a flowchart depicting a method 400 executed by the control module 308 of the phototherapy blanket 300.

Once the LEDs 302 are switched ON and while the treatment is provided to the baby enveloped in the blanket 300, the control module 308 checks constantly the temperature, more specifically, the temperature of two regions over the temperature sensor(s) 304 inside the blanket 300.

At step 402, the control module 308 receives a first signal. The first signal is indicative of the temperature of the region laying over the temperature sensor 304. In the current embodiment, the control module 308 receives two first signals from each of the temperature sensors 304. In particular the control module 308 checks if the recorded temperature (T) from one of the temperature sensors is greater than a temperature threshold (T_{threshold}). If at 402, it is determined by the control module 308 that the T is not greater than T_{threshold}, then the LEDs 302 are continued to be switched ON and the state of the LEDs 302 is thus maintained as ON and the treatment is continued. In another embodiment of the invention, once the control module 308 continues the state of the LEDs 302 to remain ON, the control module 308 can further control the intensity of the LEDs 302, such as to increase the luminosity/ brightness of the LEDs 302.

However at step 402, if it is determined, that T is greater than Threshold, then the control module 308 further receives a second signal from the surface sensor 306 at step 404. In an embodiment of the invention, the control module checks at step 404, if the recorded temperature is indeed because of the region of the surface, in particular skin, of the baby. In current embodiment, the photo-resistor 306 adjacent to the temperature sensor 304, is used to determine if the temperature recorded is because of the region of the surface (skin) of the baby. It is well known in the art that the photo-resistor responds to the optical energy incident on it. Thus, if the baby is not lying over the thermistor 304, then the adjacent photo-resistor 306 faces LEDs 302 in the front surface 301a and outputs a corresponding temperature that is recorded because of the optical energy incident on the photo-resistor 306. Thus, recording of the temperature by the photo-resistor 306 is interpreted as "non-detection" of the region. To further elaborate, if the baby was lying above the thermistor 304, then the photo-resistor 306 would not face (as is thus occluded from) the LEDs 302 arranged on the front surface 301 and thus will not encounter any optical energy and hence will produce the second signal indicative of no recorded temperature and which is interpreted as detection of the region.

Hence, if at the step 404 it is determined by the control module 308, that a second signal indicative of a temperature is provided by the photo-resistor 306, then control module 308 continues to retain the state of the LEDs 302 as ON at the step 406. Additionally, an alarm signal indicating that the baby has moved out of the treatment area, may be given to the caregiver.

However, if the skin is detected, i.e. there is no indication of the temperature in the signal provided by the photo-resistor 306, then the control module 308 switches OFF LEDs 302 at step 406. Such a determination confirms that the temperature recorded by the thermistor 304 is indeed because of the skin of the baby. It may be apparent to a person skilled in the art that both ON and OFF states are also form of controlling the LEDs 302.

Further, as an optional step 408, once the LEDs 302 are switched OFF, the control module 308 sends an alert signal to a caregiver of the baby. This helps to cater to the high temperature of the baby immediately.

In an alternate embodiment of the invention, the photo-resistor 306 can be replaced by a photodiode/photodetector (not shown). As known in the art the photodiodes are configured to provide a signal (voltage output) corresponding to the light incident on it. Thus after the step 402 is executed as explained above, the control module 308 checks at steps 404, if a second signal (voltage output) is provided by the photo-diode. In the current example, if the photo-diode provides the second signal that is indicative of light incident on it, then the second signal is interpreted as "non-detection" of the region of the surface. Consequently, if the second signal is indicative of no light output, then control module 308 interprets it as "region detection". Similar steps (406, 408), as explained in the previous embodiment, can be once the non-detection is confirmed.

In both the embodiments above, "non-detection of region" is interpreted from the second signal indicative of a light output, either temperature or voltage. In absence of such an information, the control module 308 interprets the absence as "region detection".

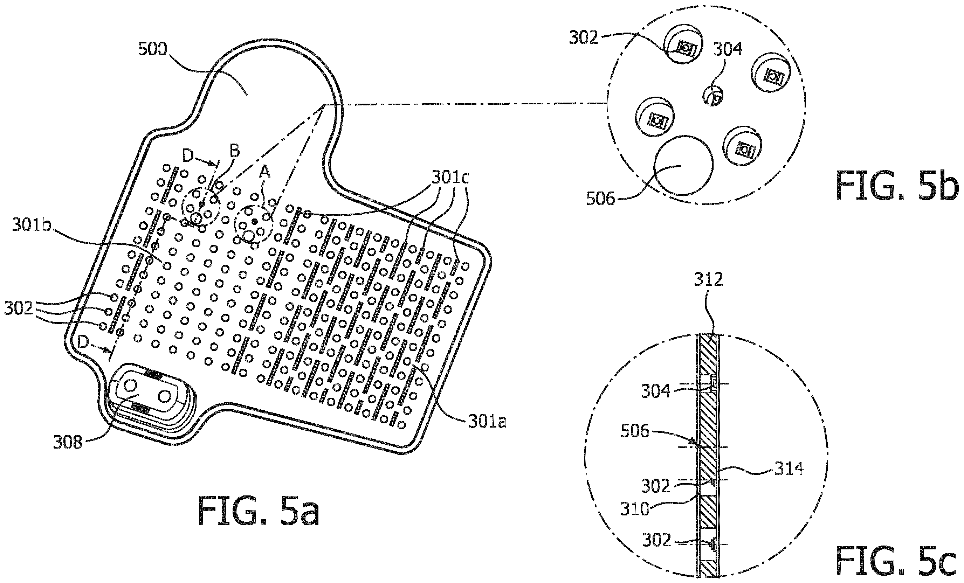

Figure 5a, 5b, and 5c shows a phototherapy blanket 500 according to another embodiment of the invention.

Similar reference numbers as in Fig. 3 refer to same components and thus have same functionality. Further working of this embodiment will be explained in the conjunction with Fig. 4. The only difference with respect to the embodiment explained in Fig. 3 is that the "region-detection" is interpreted as a direct contact of the baby with a surface sensor 506, such as skin conductance senor 506, capacitive sensor (not shown), pressure sensor (not shown) or a combination thereof. Such sensors can be thus termed as contact sensors primarily because they provide a signal output indicative of contact only if the baby contacts the sensor directly.

In the current set up, the once the control module 308 executes steps 402, it proceeds to step 404 to receive the second signal indicative of presence of the region. In the current embodiment, the presence of the region is determined by one of the contact sensors 306 placed in proximate to the temperature sensor 304 that recorded the temperature. Thus, once it is determined that the baby is in contact, i.e. "skin detection", similar steps (406, 408), as explained in the previous embodiment (Fig. 4), can be executed once the "skin detection" is confirmed.

Similarly, the capacitive sensor (not shown) can provide a second signal indicative of touch, and hence indicative of "skin detection". Also, the pressure sensor (not shown) can provide a signal indicative of pressure distribution around/ in vicinity of the temperature sensor, and hence indicative of "skin detection". In the embodiment of the pressure sensor, the thermistor can lay directly above the pressure sensor and need not be necessarily placed next to it. Thus, proximity need not be only construed in only in one dimension/plane.

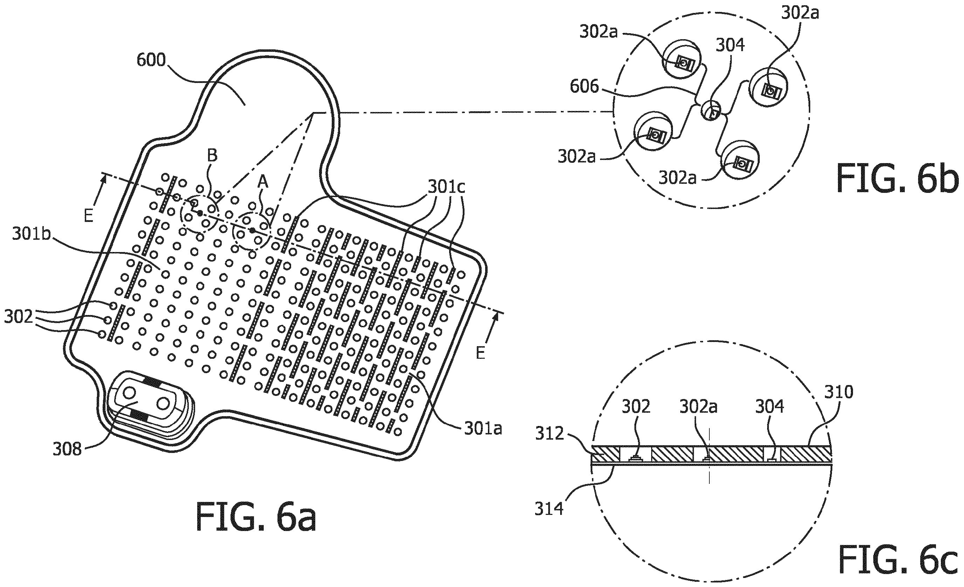

Figure 6a, 6b, and 6c shows a phototherapy blanket 600 according to another embodiment of the invention. Similar reference numbers as in Fig. 3 refer to same components and thus have same functionality. Further working of this embodiment will be explained in the conjunction with Fig. 4.

The difference with respect to the previous embodiments is that the LEDs in vicinity, preferably surrounding, of the thermistor 304 act as a surface sensor. This is further explained below.

Like in the previous embodiments, once the control module 308 executes step 402, it proceeds to step 404 to receive the second signal that provides information on the presence of the region of the surface above the temperature sensor 304. In the current set up, the control module switches the LEDs 302a in a detection mode, for a time period, around the thermistor 304. It is well known that LEDs, when not emitting light, can act as photo-diodes/ photo detectors. In other words, they provide a voltage output corresponding to the incident light. Thus, the control module 308, like in the embodiment in the explained in Fig. 3, checks for a second signal (voltage output) indicative of light detection, which is then interpreted as "non-detection" of the region of the surface of the body part. Consequently, if the second signal is indicative of no light output, then control module 308 interprets it as "region detection". Thus, a set of LEDs 302a of the LEDs 302 in the blanket 600 are used as the surface sensor 606. In the current example, though four LEDs 302a are used to detect light, it may be apparent to a person skilled in the art, that one or more of such LEDs can be used to for detecting incident light. Thereafter, similar steps 406 and 408 can be executed based on the detection of the presence of the region.

The control module 308 includes a processor and a memory. The processor is a hardware device for executing software that can be stored in a memory. The processor can be virtually any custom made or commercially available processor, a central processing unit (CPU), a digital signal processor (DSP), or an auxiliary processor among several processors associated with a computer, and the processor may be a semiconductor based microprocessor (in the form of a microchip) or a microprocessor.

In various previous embodiments explained above, the temperature threshold is a pre-determined threshold. As per the standard guidelines, the temperature must not exceed 37.5 +- 1.5 degree Celsius. In an alternative embodiment, the temperature threshold can be dynamically determined based on certain input parameters. In yet another embodiment, of the invention, first signal may be indicative of an average temperature over a time-period and the control module 308 checks for the second signal when the average temperature and the time-period deviate from a pre-defined correlation. These thresholds and/ or correlation and/ or an algorithm to dynamically determine the temperature threshold can be stored in memory (not shown in the figures).

The memory can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory may incorporate electronic, magnetic, optical, and/or other types of storage media. Note that the memory can have a distributed architecture, where various components are situated remote from one another, but can be accessed by the processor.

The software in the memory may include one or more separate programs, each of which comprises an ordered listing of executable instructions for implementing logical functions. The software in the memory for example may include one or more of a suitable operating system (O/S), compiler, source code, and one or more applications in accordance with exemplary embodiments.

The invention is defined in claim 1. Further aspects and preferred embodiments are defined in the dependent claims. Aspects, embodiments and examples of the present disclosure which do not fall under the scope of the appended claims do not form part of the claimed invention and are merely provided for illustrative purposes.

## Claims

1. A phototherapy blanket (300,500,600) configured to cover, support, and/or envelop at least part of a subject, the phototherapy blanket comprising:
a set of light sources (302) configured to emit electromagnetic radiation, wherein the set of light sources is held and/or carried by the phototherapy blanket;
a temperature sensor (304) configured to generate a first signal conveying information related to a temperature of a region of a surface of the at least part of the subject covered, supported, and/or enveloped in the phototherapy blanket;
a surface sensor (306, 506, 606) configured to provide information about presence of the surface of the at least part of the subject, wherein the surface sensor is positioned proximate to the temperature sensor such that the surface sensor is configured to generate a second signal indicative of presence of the region of the surface of the at least part of the subject; and
a control module (308) configured to control the intensity of the set of light sources based on the first and the second signal, **characterized in that** the control module (308) is further configured to determine if the temperature of the region is above a temperature threshold, wherein when the temperature of the region is above the temperature threshold, the control module is further configured to control the intensity of the set of light sources based on the second signal.

2. The phototherapy blanket according claim 1, wherein the temperature sensor (304) and surface sensor (306, 506, 606) are placed in a pair, wherein the phototherapy blanket comprises at least two such sensor pairs, wherein when the control module determines that the temperature of the region is above a temperature threshold based on the first signal provided by a first temperature sensor of a first sensor pair of the at least two sensor pairs, the control module is further configured to control the intensity of the set of light sources based on the second signal provided by the corresponding first surface sensor of the first sensor pair.

3. The phototherapy blanket according to any of the previous claims, wherein controlling the intensity comprises maintaining an ON state of the light sources.

4. The phototherapy blanket according to any of the previous claims, wherein controlling the intensity further comprises switching OFF the light sources.

5. The phototherapy blanket according to claim 4, wherein the control module (308) is further configured to generate an alert for a caregiver of the subject in the OFF state.

6. The phototherapy blanket according to claim 1, wherein the surface sensor (306) is at least one of a photo resistor or a photodiode.

7. The phototherapy blanket according to claim 6, wherein when the temperature of the region is above a temperature threshold, the control module is configured to check if the second signal that the surface sensor outputs is indicative of light, wherein when the second signal indicates that light is detected, the second signal is indicative of non-detection of the region of the surface of the at least part of the subject.

8. The phototherapy blanket according to claim 1, wherein the surface sensor (506) is one of a skin conductance sensor, a pressure sensor and a capacitive sensor.

9. The phototherapy blanket according to claim 1, wherein the light source is an LED (302), and wherein the control module is configured to switch at least one LED in proximate to the temperature sensor in a detection mode for a time-period, wherein, the LED in the detection mode is the surface sensor (606), wherein the LED in detection mode is a photodetector and the control module is further configured to check if the second signal that the photodetector outputs is indicative of light, wherein when the second signal indicates that light is detected, the second signal is indicative of non-detection of the region of the surface of the at least part of the subject.

10. The phototherapy blanket according to claim 1, wherein the temperature sensor (304) is a thermistor.

11. A computer program product comprising computer program code means for implementing a method, wherein the method is performed when said program is run by a control system of a phototherapy blanket as claimed in claim 1, the phototherapy blanket comprising a set of light sources, the method comprising the following steps:
receiving (402) a first signal indicative of a temperature of a region of a surface of at least part of a subject; wherein the at least part of the subject is covered and/ or enveloped in the phototherapy blanket, wherein the first signal is provided by a temperature sensor included in the phototherapy blanket;
receiving (404) a second signal indicative of presence of the region of a surface of the at least part of the subject, wherein the second signal is provided by a surface sensor, wherein the surface sensor is positioned in proximate of the temperature sensor; and
controlling (406) the intensity of the set of light sources based on the first and the second signal, **characterized in that** the method further comprises the step of determining if the temperature of the region is above a temperature threshold, wherein when the temperature of the region is above the temperature threshold, the method further comprises the step of controlling the intensity of the set of light sources based on the second signal.

12. The computer program product according to claim 11 further comprising the step of controlling the intensity of the light sources comprises switching OFF the light sources.

13. The computer program product according to claim 12 further comprising the step of generating an alert for a caregiver of the subject in the OFF state.

## Patentansprüche

1. Phototherapie-Decke (300, 500, 600), die dazu ausgelegt ist, mindestens einen Teil einer Person abzudecken, zu unterstützen und/oder
zu umhüllen, wobei die Phototherapie-Decke Folgendes umfasst:
einen Satz von Lichtquellen (302), die dazu ausgelegt sind, elektromagnetische Strahlung auszusenden,
wobei der Satz von Lichtquellen von der Phototherapie-Decke gehalten und/oder getragen wird;
einen Temperatursensor (304), der dazu ausgelegt ist, ein erstes Signal zu erzeugen, das Informationen in Bezug auf eine Temperatur eines Bereichs einer Oberfläche der zumindest teilweise von der Phototherapie-Decke bedeckten, unterstützten und/oder umhüllten Person zu übermitteln;
einen Oberflächensensor (306, 506, 606), der dazu ausgelegt ist, Informationen über das Vorhandensein der Oberfläche zumindest eines Teils der Person zu liefern, wobei der Oberflächensensor in der Nähe des Temperatursensors positioniert ist, sodass der Oberflächensensor so konfiguriert ist, dass er ein zweites Signal erzeugt, das das Vorhandensein des Bereichs der Oberfläche des zumindest einen Teils der Person anzeigt; und
ein Steuermodul (308), das so konfiguriert ist, dass es die Intensität des Satzes von Lichtquellen auf Grundlage des ersten und des zweiten Signals steuert, **dadurch gekennzeichnet, dass** das Steuermodul
(308) ferner dazu ausgelegt ist, zu bestimmen, ob die Temperatur des Bereichs über einem Temperaturschwellenwert liegt, wobei, wenn die Temperatur des Bereichs über dem Temperaturschwellenwert liegt, das Steuermodul ferner so konfiguriert ist, dass es die Intensität des Satzes von Lichtquellen auf Grundlage des zweiten Signals steuert.

2. Phototherapie-Decke nach Anspruch 1, wobei der Temperatursensor (304) und der Oberflächensensor (306, 506, 606) als Paar angeordnet sind, wobei die Phototherapie-Decke
mindestens zwei solche Sensorpaare umfasst, wobei, wenn das Steuermodul
auf Grundlage des ersten Signals, das von einem ersten Temperatursensor eines ersten Sensorpaars der mindestens zwei Sensorpaare bereitgestellt wird, feststellt, dass die Temperatur des Bereichs über einem Temperaturschwellenwert liegt, das Steuermodul ferner dazu konfiguriert ist, die Intensität des Satzes von Lichtquellen auf Grundlage des zweiten Signals, das von dem entsprechenden ersten Oberflächensensor des ersten Sensorpaars bereitgestellt wird, zu steuern.

3. Phototherapie-Decke nach einem der vorhergehenden Ansprüche, wobei die STEUERUNG der Intensität die Aufrechterhaltung eines eingeschalteten Zustandes der Lichtquellen umfasst.

4. Phototherapie-Decke nach einem der vorhergehenden Ansprüche, wobei die Steuerung der Intensität weiterhin das AUSSCHALTEN der Lichtquellen umfasst.

5. Phototherapie-Decke nach Anspruch 4, wobei das Steuermodul (308) ferner so konfiguriert ist, dass im AUSGESCHALTETEN Zustand ein Alarm für eine Pflegekraft der Person erzeugt wird.

6. Phototherapie-Decke nach Anspruch 1, bei der der Oberflächensensor
(306) ein Fotowiderstand und/oder eine Fotodiode ist.

7. Phototherapie-Decke nach Anspruch 6, wobei, wenn die Temperatur
des Bereichs über einem Temperaturschwellenwert liegt, das Steuermodul so konfiguriert ist, dass es prüft, ob das zweite Signal, das der Oberflächensensor ausgibt, Licht anzeigt, wobei, wenn das zweite Signal anzeigt, dass Licht erfasst wird, das zweite Signal die Nicht-Erfassung des Bereichs der Oberfläche zumindest eines Teils der Person anzeigt.

8. Phototherapie-Decke nach Anspruch 1, bei der der Oberflächensensor
(506) aus einem Hautleitfähigkeitssensor, einem Drucksensor und einem kapazitiven Sensor besteht.

9. Phototherapie-Decke nach Anspruch 1, wobei die Lichtquelle eine LED (302) ist, und wobei das Steuermodul so konfiguriert ist, dass es mindestens eine LED in der Nähe des Temperatursensors für eine Zeitdauer in einen Detektionsmodus schaltet, wobei die LED im Detektionsmodus der Oberflächensensor (606) ist, wobei die LED im Detektionsmodus ein Fotodetektor ist und das Steuermodul ferner so konfiguriert ist, dass es prüft, ob das zweite Signal, das der Fotodetektor ausgibt, Licht anzeigt, wobei, wenn das zweite Signal anzeigt, dass Licht detektiert wird, das zweite Signal die Nicht-Detektion des Bereichs der Oberfläche des zumindest einen Teils der Person anzeigt.

10. Phototherapie-Decke nach Anspruch 1, wobei der Temperatursensor (304) ein Thermistor ist.

11. Computerprogramm
mit Computerprogramm-Code zur
Implementierung eines Verfahrens, wobei das Verfahren durchgeführt wird, wenn das Programm von einem Steuersystem einer Phototherapie-Decke nach Anspruch 1 ausgeführt wird,
wobei die Phototherapie-Decke einen Satz Lichtquellen umfasst,
wobei das Verfahren aus folgenden Schritten besteht:
Empfang (402) eines ersten Signals, das eine Temperatur eines Bereichs einer Oberfläche mindestens eines Teil einer Person anzeigt; wobei der mindestens eine Teil der Person von der Phototherapie-Decke bedeckt und/oder eingehüllt ist, wobei das erste Signal von einem in der Phototherapie-Decke enthaltenen Temperatursensor bereitgestellt wird;
Empfang (404) eines zweiten Signals, das das Vorhandensein des Bereichs einer Oberfläche des zumindest einen Teils der Person anzeigt, wobei das zweite Signal von einem Oberflächensensor bereitgestellt wird, wobei der Oberflächensensor in der Nähe des Temperatursensors positioniert ist; und
Steuerung (406) der Intensität des Satzes von Lichtquellen auf Grundlage der ersten und des zweiten Signals, **dadurch gekennzeichnet, dass** das Verfahren ferner den Schritt des Bestimmens umfasst, ob die Temperatur des Bereichs über einer Temperaturschwelle liegt, wobei, wenn die Temperatur des Bereichs oberhalb der Temperaturschwelle liegt, das Verfahren ferner den Schritt der
Steuerung der Intensität des Satzes von Lichtquellen auf Grundlage des zweiten Signals umfasst.

12. Computerprogramm nach Anspruch 11, wo ferner den Schritt der Steuerung der Intensität
der Lichtquellen das AUSSCHALTEN der Lichtquellen umfasst.

13. Computerprogramm nach Anspruch 12, das ferner im AUSGESCHALTETEN Zustand den Schritt des Erzeugens eines Alarms für eine Pflegekraft der Person umfasst.

## Revendications

1. Couverture de photothérapie (300, 500, 600) conçue pour couvrir, soutenir et/ou envelopper au moins une partie d'un sujet, ladite couverture de photothérapie comprenant :
un ensemble de sources lumineuses (302) conçu pour émettre un rayonnement électromagnétique,
dans laquelle ledit ensemble de sources lumineuses est maintenu et/ou porté par la couverture de photothérapie ;
un capteur de température (304) conçu pour générer un premier signal véhiculant
des informations liées à une température d'une zone d'une surface de l'au moins une partie couverte du sujet, soutenue et/ou enveloppée dans la couverture de photothérapie ;
un capteur de surface (306, 506, 606) conçu pour fournir des informations sur
la présence de la surface d'au moins une partie du sujet, dans laquelle le capteur de surface est positionné à proximité du capteur de température de telle sorte que le capteur de surface est conçu pour générer un second signal indicatif de la présence de la zone de la surface de l'au moins une partie du sujet ; et
un module de commande (308) conçu pour commander l'intensité de l'ensemble de sources lumineuses en fonction du premier et du second signal, **caractérisé en ce que** le module de commande (308) est en outre conçu pour déterminer si la température de la zone est supérieure à
un seuil de température, dans laquelle lorsque la température de la zone est supérieure au seuil de température, le module de commande est en outre conçu pour commander l'intensité de l'ensemble de sources lumineuses en fonction du second signal.

2. Couverture de photothérapie selon la revendication 1, dans laquelle le capteur de température (304) et le capteur de surface (306, 506, 606) sont placés dans une paire, dans laquelle la couverture de photothérapie
comprend au moins deux desdites paires de capteurs, dans laquelle lorsque le module de commande détermine que la température de la zone est supérieure à un seuil de température en fonction du premier signal fourni par un premier capteur de température d'une première paire de capteurs des au moins deux paires de capteurs, le module de commande est en outre conçu pour commander l'intensité de l'ensemble de sources lumineuses en fonction du second signal fourni par le premier capteur de surface correspondant de la première paire de capteurs.

3. Couverture de photothérapie selon l'une quelconque des revendications précédentes, dans laquelle la commande de l'intensité comprend le maintien d'un état de MARCHE des sources lumineuses.

4. Couverture de photothérapie selon l'une quelconque des revendications précédentes, dans laquelle la commande de l'intensité comprend en outre la mise en ARRÊT des sources lumineuses.

5. Couverture de photothérapie selon la revendication 4, dans laquelle le module de commande (308) est en outre conçu pour générer une alerte pour un soignant du sujet dans l'état d'ARRÊT.

6. Couverture de photothérapie selon la revendication 1, dans laquelle le capteur de surface (306) est une photorésistance et/ou une photodiode.

7. Couverture de photothérapie selon la revendication 6, dans laquelle lorsque la température de la zone est supérieure à un seuil de température, le module de commande est conçu pour vérifier si le second signal émis par le capteur de surface indique la lumière, dans laquelle lorsque le second signal indique ladite lumière détectée, le second signal indique une non-détection de la zone de la surface de l'au moins une partie du sujet.

8. Couverture de photothérapie selon la revendication 1, dans laquelle le capteur de surface
(506) est une un capteur de conductance de la peau, un capteur de pression et un capteur capacitif.

9. Couverture de photothérapie selon la revendication 1, dans laquelle la source lumineuse est une LED (302), et dans laquelle le module de commande est conçu pour commuter au moins une LED à proximité du capteur de température dans un mode de détection pendant une période de temps, dans laquelle la LED en mode de détection est le capteur de surface (606), dans laquelle la LED en mode détection est un photodétecteur et le module de commande est en outre conçu pour vérifier si le second signal émis par le photodétecteur est indicatif de la lumière, dans laquelle lorsque le second signal indique la lumière détectée, le second signal indique une non-détection de la zone de la surface de l'au moins une partie du sujet.

10. Couverture de photothérapie selon la revendication 1, dans laquelle le capteur de température (304) est une thermistance.

11. Produit de programme informatique
comprenant un moyen de code de programme informatique pour
mettre en œuvre un procédé, dans lequel le procédé est mis en œuvre lorsque ledit programme est exécuté par un système de commande d'une couverture de photothérapie selon la revendication 1,
ladite couverture de photothérapie comprenant un ensemble de sources lumineuses,
ledit procédé comprenant les étapes suivantes :
la réception (402) d'un premier signal indicatif d'une température d'une zone d'une surface d'au moins une partie d'un sujet ; dans lequel l'au moins une partie du sujet est couverte et/ou enveloppée dans la couverture de photothérapie, dans lequel le premier signal est fourni par un capteur de température inclus dans la couverture de photothérapie ;
la réception (404) d'un second signal indiquant la présence de la zone d'une surface de l'au moins une partie du sujet, dans lequel le second signal est fourni par un capteur de surface, dans lequel le capteur de surface est positionné à proximité du capteur de température ; et
la commande (406) de l'intensité de l'ensemble de sources lumineuses en fonction du premier et du second signal, **caractérisé en ce que** ledit procédé comprend en outre l'étape de
détermination si la
température de la zone est supérieure à un seuil de température, dans lequel lorsque la température de la zone est supérieure au seuil de température, ledit procédé comprend en outre l'étape de
commande de
l'intensité de l'ensemble de sources lumineuses en fonction du second signal.

12. Produit de programme informatique selon la revendication 11, comprenant en outre l'étape de commande de l'intensité
des sources lumineuses comprenant la mise en ARRÊT des sources lumineuses.

13. Produit de programme informatique selon la revendication 12, comprenant en outre l'étape de génération d'une alerte pour un
soignant du sujet dans l'état d'ARRÊT.
